# DEMANDE DE BREVET EUROPEEN

(11) **EP 2 564 831 A1**
(43) Date de publication de la demande: **06.03.2013**
(21) Numéro de dépôt: 12170593.3
(22) Date de dépôt: 25.01.2008
(51) Int. Cl.: A61K 9/107, A61K 9/06, A61K 8/06, A61Q 5/12, A61Q 5/02, A61K 8/04, A61K 8/46, A61K 8/60

(54) **Emulsion dermatologique et procédé de préparation**

(30) Priorité: 26.01.2007 FR 0752901
(62) Demande divisionnaire de: 08701673.9
(71) Demandeur: Pierre Fabre Dermo-Cosmétique, 92100 Boulogne-Billancourt (FR)
(72) Inventeur: Nielloud, Françoise, 34170 Castelnau Le Lez (FR)
(74) Mandataire: Regimbeau

(57) **Abrégé**

Composition gélifiée moussante sous forme de microémulsion huile dans eau stable, comprenant une phase grasse de microgoutelettes dispersées dans une phase aqueuse et un principe actif dermatologique, caractérisée en ce qu'elle comprend un système de tensioactifs constitué de :
- au moins un tensioactif non ionique éthoxylé ;
- au moins un cotensioactif stabilisant le tensioactif non ionique éthoxylé ;
- au moins un tensioactif non ionique hydrophile non éthoxylé ;
- au moins un agent moussant choisi dans le groupe des tensioactifs anioniques.

## Description

La présente invention concerne le domaine des formes liquides moussantes lavantes destinées au traitement des troubles dermatologiques, en particulier les troubles du cuir chevelu tels que la dermatite séborrhéique.

La dermatite séborrhéique est une affection chronique inflammatoire dermatologique affectant principalement la tête et le visage au niveau des glandes sébacées. La dermatite séborrhéique se caractérise par une desquamation marquée au niveau du cuir chevelu et à sa marge, des sourcils ainsi qu'au niveau du pli naso-labial.

Les causes de cette affection ne sont pas clairement identifiées. Des facteurs génétiques et hormonaux seraient responsables de ce phénomène. En outre, une levure lipophile du genre Pityrosporum serait impliquée dans le développement de ce trouble dermatologique ; la dermatite séborrhéique étant alors une réponse inflammatoire à ce micro-organisme.

Les traitements de la dermatite séborrhéique comprennent l'utilisation de composés antifongiques tels que les agents azolés, les goudrons, les sulfites ou le pyrithione de zinc par exemple, de manière à lutter contre l'invasion du Pityrosporum et/ou l'utilisation d'anti-inflammatoires.

Ainsi, des traitements adéquats consistent en un traitement quotidien à l'aide d'un shampooing antipelliculaire contenant du sulfite de sélénium et/ou du pyrithione de zinc. Un shampooing à base de goudrons ou contenant du kétoconazole peut aussi être utilisé. De tels shampooing doivent être appliqués sur le cuir chevelu et laissés en place environ 10 minutes avant rinçage. L'utilisation d'un shampooing hydratant ou émollient est recommandée pour limiter l'assèchement du cuir chevelu. Pour des cas plus sévères, des corticostéroïdes peuvent être utilisés en application locale.

Le brevet BE 84515 décrit une composition contenant de l'hydrocortisone dans un mélange de solvants constitué de 15 à 60% d'alcool aliphatique, 15 à 60% de propylène glycol et de 5 à 60% d'un agent solubilisant supplémentaire.

Le brevet EP 325 949 décrit une composition comprenant au moins 2,5% de corticoïdes, de 25 à 80% d'un tensioactif non ionique, de 0 à 70% d'éthanol et de 0 à 70% de propylène glycol en association avec un agent antimicrobien.

La faible fluidité de telles compositions rend leur application peu aisée, d'autant que les solvants qu'elles contiennent ont tendance à s'évaporer rapidement. Il est ainsi nécessaire de procéder à un frictionnement pour permettre la pénétration des actifs, ce qui entraîne des irritations de l'épiderme.

Pour limiter ce problème d'évaporation du solvant, la demande de brevet WO 96/27376 propose une mousse contenant un corticoïde, un agent casseur de mousse, un propulseur et un tampon. Cependant, à l'usage ce type de mousse ne permet pas d'atteindre facilement le cuir chevelu.

De tels traitements, outre leur faible agrément cosmétique, doivent être répétés sur une période de temps assez longue et s'accompagnent d'effets indésirables désagréables pour le patient tels que rougeurs, dessèchement et irritation du cuir chevelu. La lourdeur du traitement rend son observance parfois limitée.

Dans le cas de mise en oeuvre de principes actifs non hydrosolubles, leur solubilisation dans des formulations de type shampooing moussant est problématique. En outre, le temps de contact entre la formulation moussante et le cuir chevelu est limité, ce qui limite d'autant plus la pénétration cutanée de l'actif.

Ainsi, des formulations de type émulsion ou micro-émulsion huile dans eau (H/E) ou eau dans huile (E/H) ont été développées pour pallier à ce genre d'inconvénient. La présence d'une phase lipidique permet ainsi une meilleure solubilisation des principes actifs non hydrosolubles, favorisant ainsi leur pénétration cutanée. En outre, les propriétés émollientes des huiles mises en oeuvre contribuent à diminuer certains effets indésirables du traitement (assèchement). Le temps d'application de telles compositions est aussi réduit.

Le brevet US 6,607,733 décrit des compositions dermatologiques sous la forme de microémulsions H/E contenant une phase aqueuse, une phase grasse discontinue, un système stabilisant associant des émulsifiants polyéthoxylés et polypropoxylés avec des substances pontantes réalisant une liaison entre les microgouttelettes de la phase grasse. La présence de ces agents pontants est nécessaire pour assurer la stabilité de ces émulsions dans le temps. Une telle solution est intéressante mais onéreuse et techniquement difficile à mettre en oeuvre.

La demanderesse a recherché des compositions ne présentant pas les inconvénients susmentionnés, c'est à dire une composition présentant de bonnes propriétés cosmétiques et permettant une application aisée et une bonne pénétration du principe actif.

La demanderesse a ainsi mis au point une composition moussante sous la forme d'un gel de type microémulsion huile dans eau.

La demanderesse a aussi constaté que les compositions moussantes selon la présente invention présentent les propriétés précédemment mentionnées.

Ainsi, les compositions selon la présente invention permettent l'administration du principe actif au niveau de la zone à traiter, assurent sa bonne pénétration dans le derme et présentent également des performances dermatologiques et cosmétiques tout à fait intéressantes.

Les compositions selon la présente invention permettent le traitement des affections cutanées telles que la dermite séborrhéique. Les propriétés rhéologiques des compositions selon la présente invention permettent une utilisation aisée en tout point comparable à un shampooing. En particulier, la présentation sous forme de shampooing avec pouvoir lavant et moussant permet un nettoyage des squames présentes au niveau du cuir chevelu et des cheveux. En outre, les compositions selon la présente invention laissent les cheveux doux et souples et ne sont pas irritantes pour le cuir chevelu.

Il a également été constaté que les compositions selon la présente invention sont stables dans le temps et permettent d'éviter la précipitation du principe actif ou une séparation des phases de l'émulsion.

Un des objets de la présente invention est donc une composition gélifiée moussante de type microémulsion huile dans eau pour le lavage et le traitement du cuir chevelu et des cheveux.

Enfin, un autre objet de la présente invention est aussi l'utilisation des compositions selon l'invention comme médicament et pour la fabrication d'un médicament destiné à traiter les affections dermatologiques de la peau et du cuir chevelu.

L'invention concerne donc plus précisément une composition gélifiée moussante sous forme de microémulsion huile dans eau stable, comprenant une phase grasse de microgoutelettes dispersées dans une phase aqueuse et un principe actif dermatologique, caractérisée en ce qu'elle comprend un système de tensioactifs constitué de :
- au moins un tensioactif non ionique éthoxylé ;
- au moins un cotensioactif stabilisant le tensioactif non ionique éthoxylé ;
- au moins un tensioactif non ionique hydrophile non éthoxylé ;
- au moins un agent moussant choisi dans le groupe des tensioactifs anioniques.

Au sens de la présente invention, on entend par "cotensioactif stabilisant le tensioactif non ionique éthoxylé", un tensioactif se plaçant entre les autres molécules de tensioactif et apportant de la souplesse et de la fluidité aux films interfaciaux, permettant ainsi de stabiliser les films interfaciaux entre l'huile et l'eau.

Au sens de la présente invention, on entend par "agent moussant", un tensioactif pouvant se placer à l'interface eau-air, ceci ayant pour effet de stabiliser la mousse formée.

Avantageusement, la composition selon l'invention ne contient pas d'agent pontant. On entend par "agent pontant" une molécule comportant au moins deux régions capables d'intéragir avec les goutelettes d'huile par une intéraction hydrophobe, formant ainsi un pont entre deux ou plusieurs goutelettes d'huile, et une région intéragissant avec la phase aqueuse par une intéraction hydrophile.

Le principe actif contenu dans la composition selon la présente invention est un principe actif dermatologique, doté de vertus thérapeutiques vis à vis des affections cutanées de la peau et/ou du cuir chevelu.

Dans le cadre de la présente invention, le principe actif dermatologique est, par exemple, un principe actif non hydrosoluble préférentiellement solubilisé dans la phase grasse de la microémulsion.

Les corticoïdes sont particulièrement bien adaptés en tant que principe actif dermatologique selon la présente invention. Les corticoïdes de la présente invention peuvent être choisis dans le groupe comprenant le dipropionate d'alclométasone, l'amcinonide, le dipropionate de beclométhasone, le benzoate de béthamethasone, valérate de betaméthasone, le dipropionate de béthaméthasone, le valérate de béthaméthasone, le budesonide, le propionate de clobétasol, préférentiellement le 17 propionate de clobétasol, le butyrate de clobétasol, le désonide, la désoximétasone, la dexaméthasone, le diacétate dediflorasone, le valérate de diflucortolone, la flurandrénolone, l'acétate de fluprednidene, le fluocortolone, le butyle de fluocortine, le fluocinonide, l'acétonide de fluocinolone, l'acétonide de fluclorolone, le pyvalate de flumétasone, le chlorhydrate de feudiline, la flumétholone, l'halcinonide, l'hydrocortisone, l'acétate d'hydrocortisone, le butyrate d'hydrocortisone, le valérate d'hydrocortisone, l'acétate de méthylprednisolone, le furoate de mométasone, la methylprednisolone, la prednisolone, l'acétonide de triamcinolone ou parmi des mélanges pharmaceutiquement acceptables de ces derniers.

Le principe actif est de manière avantageuse le valérate de bétaméthasone.

Les principes actifs antifongiques présentent un intérêt dans le cadre de la présente invention.

Avantageusement, les antifongiques sont choisis dans le groupe comprenant les imidazoles, les pyridones non imidazolées, les morpholines, les allylamines.

L'imidazole peut avantageusement être choisi dans le groupe comprenant le kétoconazole, le bifonazole, le clotrimazole, l'éconazole, le fenticonazole, l'isoconazole, le miconazole, l'omoconazole, l'oxyconazole, le sulconazole, le tioconazole.

La pyridone non imidazolée est avantageusement la cyclopiroxolamine.

Avantageusement, la morpholine est l'amorolfine.

L'allylamine peut être choisie dans le groupe comprenant la terbinafine et la tolnaftate.

Les principes actifs anti-séborrhéiques, tels que par exemple le sulfure de sélénium et le peroxyde de benzoyle, peuvent être utilisés selon la présente invention.

La quantité de principe actif dermatologique est comprise en poids entre 0,001% et 5%, avantageusement entre 0,01% et 1%, plus avantageusement encore entre 0,05% et 1%, encore plus avantageusement environ égale à 0,1% du poids total de la composition.

La phase aqueuse peut contenir, outre l'eau, des solvants cosmétiquement acceptables tels que par exemple le glycérol, le propylène glycol, le sorbitol, l'éthanol, les polyéthylène glycols, l'isopropanol, un ou plusieurs composés hydrosolubles comme des acides ou des bases, des colorants, des agents actifs hydratants, apaisants, cicatrisants, exfoliants, antioxydants ou tout autre additif ou adjuvant non préjudiciable à la stabilité des microémulsions.

La phase aqueuse peut également contenir un ou plusieurs composés hydrosolubles comme des acides ou des bases, des colorants, des agents actifs hydratants, apaisants, cicatrisants, exfoliants, antioxydants ou tout autre additif ou adjuvant non préjudiciable à la stabilité des microémulsions.

La quantité de phase aqueuse de la composition selon la présente invention est avantageusement comprise en poids entre 30% et 60%, plus avantageusement entre 35% et 55% du poids total.

La phase grasse de la composition revêt toute son importance dans le cadre de la présente invention. En effet, les microgouttelettes de cette phase grasse (phase discontinue de la microémulsion) servent à solubiliser le principe actif non hydrosoluble et à véhiculer ce dernier au niveau du cuir chevelu, assurant ainsi sa vectorisation dans le derme. Ainsi, la biodisponibilité de la composition selon la présente invention est très améliorée par rapport à une composition dont le principe actif se trouverait sous la forme de particules dispersées avec un passage transcutané très limité.

En outre, comme indiqué dans l'introduction de la présente demande, les problèmes de sécheresse cutanée rencontrés avec les compositions de l'art antérieur peuvent être limités ou inhibés. En effet, la phase grasse de la composition selon l'invention présente les propriétés émollientes permettant de préserver la peau et le cuir chevelu.

La quantité de phase grasse dans la composition selon la présente invention est avantageusement comprise en poids entre 0,5% et 20%, plus avantageusement entre 2% et 18%, plus avantageusement encore entre 2,5% et 15%, et encore plus avantageusement entre 5% et 10% du poids total de la composition.

Les composants de la phase grasse peuvent être choisis dans le groupe comprenant les huiles végétales ou minérales, les triglycérides, les monoglycérides, les diglycerides, les dérivés d'acides gras, les esters d'acides gras, les huiles naturelles hydrogénées ou non, synthétiques ou non, hydrocarbonées, cycliques ou aliphatiques, linéaires ou ramifiées (saturées ou non), les silicones volatiles ou non, organomodifiées ou non, solubles ou non, des huiles perfluorées ou fluorées, les polybutènes et polyisobutènes, des esters gras se présentant sous forme liquide, utilisés seuls ou en mélange. Par exemple, il peut s'agir d'une huile de paraffine, de myristate d'octododécyle, de myristate d'isopropyle, de tricaprilic glycérol, de capric-caprilique glyceride, utilisés seuls ou en mélange.

On entend par silicone organomodifiée une silicone comportant un ou plusieurs groupements fonctionnels fixés sur la chaine siloxanique, éventuellement par l'intermédiaire d'un radical hydrocarboné. On cite à titre d'exemple de tels groupements les polyéthylènoxy, les polypropylènoxy comportant éventuellement des groupes alkyles tel que l'alkyle méthicone copolyol vendue par la société Dow Corning sous la dénomination Q2 5200.

Le tensioactif non ionique éthoxylé peut être choisi dans le groupe comprenant les alcools gras éthoxylés, les acides gras éthoxylés, les acides gras éthoxylés ethérifiés par des groupes alkyle ou alkényle, les acides gras éthoxylés estérifiés par des groupes alkyle ou alkényle, les esters éthoxylés d'acides gras saturés ou insaturés, de glycérol, de propylène glycol, de glycol, de polyéthylène glycol, de sorbitan, les mélanges de glycérides et d'esters capriliques de polyéthylène glycol, des copolymères d'oxyde d'éthylène et d'oxyde de propylène (type Poloxamer® ou Pluronic®).

La quantité de tensioactif non ionique éthoxylé présent dans la composition selon la présente invention est avantageusement inférieure en poids à 20% du poids total de la composition, plus avantageusement comprise entre 10% et 20% du poids total.

Afin de stabiliser la microémulsion selon la présente invention, la présente composition comprend au moins un cotensioactif stabilisant le tensioactif non ionique éthoxylé. La quantité de stabilisant sera définie par rapport au tensioactif non ionique éthoxylé. Le ratio cotensioactif: tensioactif non ionique éthoxylé peut avantageusement être compris en poids entre 1:2 et 1:6, plus avantageusement entre 1:2 et 1:4, ceci selon la nature tant du cotensioactif que du tensioactif non ionique éthoxylé.

La quantité totale de tensioactif non ionique éthoxylé et de cotensioactif stabilisant est avantageusement comprise en poids entre 15% et 40%, plus avantageusement entre 20% et 35% du poids total de la composition.

Le cotensioactif stabilisant le tensioactif non ionique éthoxylé dans une composition selon la présente invention peut être choisi dans le groupe comprenant les esters non ethoxylés d'acides gras saturés ou insaturés et de glycérol, de polyglycérol, de propylène glycol, de glycol, de polyéthylène glycol ou de sorbitan, tel que le diisostéarate de triglycérol, l'oléate de triglycérol, le propylène glycol monolaurate, le propylène glycol monocaprylate, le monostéarate de sorbitan.

La quantité en poids de tensioactif non ionique hydrophile non éthoxylé est avantageusement comprise en poids 1 et 30%, plus avantageusement entre 10% et 30%, plus avantageusement encore entre 13% et 25% du poids total.

Les tensioactifs non ioniques hydrophiles non éthoxylés convenables pour la mise en oeuvre de la présente composition peuvent être choisis parmi les tensioactifs dérivés des sucres, les esters d'acide gras et de sucrose, de saccharose ou de glucose.

Avantageusement, les tensioactifs non ioniques hydrophiles non éthoxylés peuvent être choisis dans le groupe comprenant les dérivés glucosides tels que les alkylglucosides et alkylpolyglucosides, présentant des chaînes carbonées de C₈ à C₂₂ avec des nombres d'unités glucoses de 1 à 7, par exemple, octylglucoside, décylglucoside, lauryglucoside, myristylglucoside, palmitylglucoside, stéarylglucoside, oléylglucosides, behenylglucosides ; en particulier les dérivés glucosides présentant des chaînes carbonées de C₁₀ à C₁₄ avec un nombre d'unités glucose de 1 à 3 comme le décylglucoside (Oramix NS10, Seppic).

Le pH des compositions selon la présente invention est avantageusement compris entre 3 et 8, plus avantageusement entre 4 et 6. Le choix du pH sera fonction du principe actif retenu et de sa structure chimique.

Le pH est avantageusement ajusté par l'ajout d'acide lactique.

La composition selon la présente invention comprend aussi un agent moussant anionique, afin de renforcer la fonctionnalité moussante et nettoyante utile pour les applications souhaitées de la présente invention. L'agent moussant selon la présente invention peut être choisi dans le groupe des tensioactifs anioniques comprenant les sels de sodium, d'ammonium, d'amines, d'aminoalcools ou de magnésium des composés suivants : les alkylsulfates, les alkyléthersulfates, les alkylamidoéthersulfates, les alkylarylpoly-éthersulfates, les alkyle monoglycérides sulftates, les alkyle monoglycérides éther sulfates, les alkyle monoglycérides sulfonates, les alkylsulfonates, les alkylphosphates, les alkyle isethionates, les alkylcarboxylates, les alkyle éther carboxylates, les alkyle éther amidocarboxylates, les alkylamidesulfonates, les alkylarylsulfonates, les a-oléfine-sulfonates, les paraffine-sulfonates, les alkylsulfosuccinates, les alkyléthersulfosuccinates, les alkylamidesulfosuccinates, les alkylsulfosuccinamates, les alkylsulfoacétates, les alkylétherphosphates, les acylsarcosinates, les N-acyle aminoacides, les acyliséthionates et les N-acyltaurates.

Avantageusement, la quantité en poids d'agent moussant anionique est comprise entre 15% et 25% du poids total.

La composition selon la présente invention peut en outre comprendre des adjuvants traditionnellement utilisés dans les compositions dermatologiques ou cosmétiques comme les shampooings. La présente composition pourra ainsi comprendre des agents stabilisants ou renforçateurs de mousse tels que les amides d'acides gras, alkylamides, mono et diéthanolamides, isopropanolamides d'acides gras.

Avantageusement, l'agent stabilisant ou renforcateur de mousse sera le diéthanolamide d'acide gras de coco, par exemple le Comperlan KD (Cognis).

La quantité d'agent stabilisant ou renforçateur de mousse dans la présente composition peut avantageusement être comprise en poids entre 0% et 10%, avantageusement entre 2% et 8%, par exemple 6% en poids par rapport au poids total de la composition.

La composition selon la présente invention se présente ainsi sous la forme d'une microémulsion huile dans eau, dans laquelle le principe actif dermatologique non hydrosoluble se retrouve au niveau de la phase grasse. Cette phase grasse est stabilisée grâce au système de tensioactifs et de cotensioactif mis en oeuvre dans la présente composition.

De manière à gélifier la composition selon la présente invention pour une meilleure praticité d'usage, cette composition pourra contenir des agents épaississants choisis notamment parmi des agents non pontants tels que les électrolytes (avantageusement le chlorure de sodium ou le citrate de sodium), les carbomères ou les gommes xanthanes.

La quantité d'agent épaississant dans la présente invention pourra être comprise en poids entre 0 et 4%, avantageusement entre 1% et 3% du poids total de la composition.

L'ajout de NaCl à la présente composition permet d'augmenter avantageusement la viscosité de la dite composition. La présente composition pourra ainsi comprendre jusqu'à 2%, avantageusement 1% de NaCl, en poids par rapport au poids total de la composition.

Ainsi, dans le cadre de la présente invention, les compositions sont sous la forme de liquides, particulièrement sous la forme de liquides visqueux.

Afin de protéger la composition selon la présente invention des risques de contamination microbienne, un ou plusieurs conservateurs peuvent être ajoutés. Les conservateurs convenables pour la mise en oeuvre de la présente composition peuvent être choisis parmi les conservateurs classiques des médicaments à usage topique tels que les acides et leurs sels (acide borique, acide benzoique, acide lactique, acide sorbique), les alcools (alcool benzylique, phénoxyéthanol, chlorocrésol), les esters de l'acide parahydroxybenzoïque (méthyle, éthyle, propyle, butyle, parabens), et les dérivés de l'imidazolidinurée. Avantageusement on choisira des conservateurs dans le groupe comprenant l'acide sorbique et ses sels, en particulier le sorbate de potassium utilisé à une concentration de 0,01 à 0,2% en poids.

Les compositions selon l'invention sont plus particulièrement utilisées comme shampooing pour le traitement du cuir chevelu. Lorsque le principe actif choisi est le valérate de bétaméthasone, la composition selon l'invention est plus particulièrement destinée au traitement de la dermatite séborrhéique de la peau en général, et du cuir chevelu en particulier.

Lors de son utilisation, la composition est appliquée sur cheveux mouillés. On procède à un léger massage au cours duquel se forme une mousse, suivi d'un rinçage soigneux après avoir respecté un temps de pose. On peut procéder à une seconde application suivie d'un nouveau rinçage soigneux à l'eau.

L'effet émollient de la composition selon la présente invention rend son utilisation particulièrement adaptée au traitement des troubles dermatologiques tels que la dermatite séborrhéique. En effet, cette affection étant caractérisée par des rougeurs et des irritations, l'utilisation de la composition selon la présente invention permet de limiter les phénomènes de sécheresse cutanée traditionnellement observés.

Les compositions selon la présente invention peuvent être fabriquées selon des techniques conventionnelles de l'industrie pharmaceutique et cosmétique.

Ainsi dans un premier temps on procèdera au mélange intime de la phase grasse avec les différents tensioactifs et le cotensioactif. Ledit mélange pourra être soumis à une homogénéisation par agitation mécanique avant l'émulsification.

La phase aqueuse contenant les éventuels adjuvants hydrosolubles tel que par exemple le NaCl sera préparée séparément, et incorporée par la suite à la phase grasse.

L'étape d'incorporation de la phase aqueuse dans la phase grasse est réalisée sous agitation, à une vitesse et pendant un temps suffisant pour atteindre la transparence de la composition.

Le pH de la microémulsion ainsi obtenue pourra être ajusté *via* l'ajout d'une solution acide ou basique. A l'issue de l'ajustement du pH, l'incorporation du principe actif pourra être réalisée par agitation jusqu'à dissolution complète à température ambiante. A titre d'exemple, dans le cas de l'utilisation de valérate de bétaméthasone, l'utilisation d'acide lactique pour ajuster le pH sera préférée.

L'invention a donc également pour objet un procédé de préparation d'une composition selon l'invention, comprenant les étapes successives suivantes :
- mélanger la phase grasse avec le système de tensioactifs et homogénéiser le mélange par agitation mécanique ;
- mélanger les différents adjuvants dans la phase aqueuse, comme par exemple l'agent épaississant ;
- incorporer la phase aqueuse dans la phase grasse sous agitation, à une vitesse et pendant un temps suffisant pour atteindre la transparence de la composition ;
- éventuellement ajuster le pH de l'émulsion par l'ajout d'une solution acide ou basique ;
- incorporer le principe actif par agitation jusqu'à dissolution complète de celui-ci à température ambiante.

L'invention a également pour objet une composition telle que précédemment décrite en tant que médicament.

L'invention a également pour objet l'utilisation d'une composition telle que précédemment décrite pour la fabrication d'un médicament destiné à traiter les troubles dermatologiques, le cuir chevelu et la dermatite séborrhéique.

L'invention a également pour objet l'utilisation d'une composition telle que précédemment décrite en tant que cosmétique.

L'invention va maintenant être illustrée, de façon non limitative, par les exemples suivants.

### Ingrédients :

- Du laureth sulfate de sodium Texapon N 70® et Texapon NSO® ont été obtenus de la société Cognis.
- De l'huile minérale Ondina 68® et Primol 352® ont été obtenues respectivement des sociétés Schell et Exxon Mobil.
- De l'huile de castor hydrogénée PEG-40 Labrasol® a été obtenue de la société Gattefosse.
- De la cocamide MEA Comperlan KD® a été obtenue de la société Cognis.
- Du décylglucoside Oramix NS 10® a été obtenu de la société Seppic.
- Du plurol oléique et du plurol diisostéarique ont été obtenus de la société Gattefosse.

### Exemple 1 :

Deux microémulsions dépourvues de principe actif (bases) ont été préparées et analysées. Leur composition sont indiquées dans le Tableau 1 et leurs analyses sont rassemblées dans le Tableau 2. Les pourcentages indiquent une concentration en poids du produit correspondant en solution dans l'eau.

Les deux émulsions sont stables, transparentes et présentent une bonne analyse sensorielle et une viscosité correcte.

**Tableau 1 : Formulation des microémulsions dépourvues de principe actif**

| **Bases** | **Ingrédients** | **Proportions** |
|---|---|---|
| **1** | Ondina 68 | 5% |
| | Labrasol/plurol diisostéarique 3/1 | 18% |
| | Décylglucoside 10% | 20% |
| | Texapon NSO 6% | 20% |
| | Comperlan KD 6% | 6% |
| | NaCl | 1% |
| **2** | Ondina 68 | 5% |
| | Labrasol/plurol oléique 2/1 | 21,5% |
| | Décylglucoside 12,5% | 25% |
| | Texapon NSO 5% | 16,5% |
| | Comperlan KD 2% | 2% |
| | NaCl | 1% |

Le pouvoir moussant est obtenu en mesurant la hauteur de mousse après un barattage de 90 secondes.

La résistance à la salissure est une mesure de la hauteur de mousse après un barattage de 90 secondes et incorporation d'un ou plusieurs ml de salissure artificielle. L'émulsion à analyser (40 ml) a été placée dans une éprouvette normalisée. La salissure est constituée de lanoline 5%, huile de paraffine 5% et dioxane qsp 100%. 1 ml de salissure a été ajouté à l'émulsion. La solution a été agitée pendant 90 secondes puis la hauteur de mousse H1 a été mesurée. Après 20 secondes de repos, 1 ml de salissure a été à nouveau ajouté, la solution a été agitée pendant 30 secondes et la hauteur de mousse H2 a été mesurée.

Le pouvoir mouillant est mesuré selon une méthode adaptée des normes AFNOR NF 73-406 et NF-73-420, consistant à mesurer le temps nécessaire à l'émulsion pour mouiller complètement un disque de coton normalisé de 3 cm de diamètre. Les mesures ont été prises pour des concentrations différentes en tensioactif et le pouvoir mouillant correspond à la concentration en tensioactif pour laquelle le temps de mouillage est de 100 secondes.

**Tableau 2 : Analyse des microémulsions dépourvues de principe actif**

| | **Base 1** | **Base 2** |
|---|---|---|
| Aspect | Jaune clair | Incolore |
| pH | 5,44 | 5,67 |
| Viscosité (Pa.s), D = 100 s⁻¹ | 0,212 | 0,220 |
| Pouvoir mouillant (g/l) | 5,0 | 4,5 |
| Pouvoir moussant (cm) | H0 = 14,9 | H0 = 15 |
| Résistance à la salissure (cm) | H2 = 0,6 | H1 = 1,1 |
| | | H2 = 0,3 |
| Quantité de mousse | Importante (départ de mousse rapide) | Très importante |
| Stabilité de la mousse | Bonne | Très bonne |
| Rincage | Bon | Bon |
| Démêlage | Très bon | Bon |

### Exemple 2 :

Deux microémulsions comprenant un principe actif ont été préparées. La viscosité a été améliorée par rapport à celle des bases par augmentation de la teneur en NaCl. Le Texapon NSO a été remplacé par le Texapon N70, le pH a été ajusté par de l'acide lactique. Les formules sont indiquées dans le Tableau 3.

**Tableau 3 : Emulsions comprenant un principe actif**

| | **Formule 1** (%) | **Formule 2** (%) | **Formule 3** (%) |
|---|---|---|---|
| Oramix NS10 | 22,73 | 18,18 | 1 |
| Oramix L30 | | | 10 |
| Texapon N70 | 7,142 | 8,57 | 30 |
| Comperlan KD | 2 | 6 | |
| Labrasol | 15,66 | 13,5 | 15 |
| Plurol oléique | 7,83 | | |
| Plurol diisotéarique | | 4,5 | 5 |
| Primol 352 | 5 | 5 | |
| HP Ondina | | | 5 |
| NaCl | 1 | 1 | |
| Eau | 38,53 | 43,01 | 34 |
| Acide lactique | 0,0125 | 0,14 | Qs pH 5,5 |
| Valérate de bethaméthasone | 0,1 | 0,1 | |
| Cyclopyroxolamine | | | 1 |
| pH sans principe actif | 5,52 | 5,65 | |
| pH avec principe actif | 5,37 | 5,47 | |
| Aspect | Microémulsion jaune transparente | Microémulsion jaune clair transparente | |

## Revendications

1. Composition gélifiée moussante sous forme de microémulsion huile dans eau stable, comprenant une phase grasse de microgoutelettes dispersées dans une phase aqueuse et un principe actif dermatologique, **caractérisée en ce qu'**elle comprend un systême de tensioactifs constitué de :
- au moins un tensioactif non ionique éthoxylé ;
- au moins un cotensioactif stabilisant le tensioactif non ionique éthoxylé ;
- au moins un tensioactif non ionique hydrophile non éthoxylé ;
- au moins un agent moussant choisi dans le groupe des tensioactifs anioniques.

2. Composition selon la revendication 1, **caractérisée en ce que** le principe actif est doté de vertus thérapeutiques vis à vis des affections cutanées de la peau et/ou du cuir chevelu.

3. Composition selon l'une quelconque des revendications précédentes, **caractérisée en ce que** le principe actif est dissous dans la phase grasse.

4. Composition selon l'une quelconque des revendications précédentes, **caractérisée en ce que** le principe actif est un corticoïde pouvant être choisi dans le groupe comprenant le dipropionate d'alclométasone, l'amcinonide, le dipropionate de beclométhasone, le benzoate de béthamethasone, le dipropionate de béthaméthasone, le valérate de béthaméthasone, le budesonide, le propionate de clobétasol, préférentiellement le 17 propionate de clobétasol, le butyrate de clobétasol, le désonide, la désoximétasone, la dexaméthasone, le diacétate dediflorasone, le valérate de diflucortolone, la flurandrénolone, l'acétate de fluprednidene, le fluocortolone, le butyle de fluocortine, le fluocinonide, l'acétonide de fluocinolone, l'acétonide de fluclorolone, le pyvalate de flumétasone, le chlorhydrate de feudiline, la flumétholone, l'halcinonide, l'hydrocortisone, l'acétate d'hydrocortisone, le butyrate d'hydrocortisone, le valérate d'hydrocortisone, l'acétate de méthylprednisolone, le furoate de mométasone, la methylprednisolone, la prednisolone, l'acétonide de triamcinolone ou parmi des mélanges pharmaceutiquement acceptables de ces derniers et est de préférence le valérate de béthaméthasone.

5. Composition selon l'une quelconque des revendications 1 à 3, **caractérisée en ce que** le principe actif est un antifongique pouvant être choisi dans le groupe comprenant les imidazoles, les pyridones non imidazolées, les morpholines, les allylamines.

6. Composition selon la revendication 5, **caractérisée en ce que** l'imidazole peut être choisi dans le groupe comprenant le kétoconazole, le bifonazole, le clotrimazole, l'éconazole, le fenticonazole, l'isoconazole, le miconazole, l'omoconazole, l'oxyconazole, le sulconazole, le tioconazole.

7. Composition selon la revendication 5, **caractérisée en ce que** la pyridone non imidazolée est la cyclopiroxolamine.

8. Composition selon la revendication 5, **caractérisée en ce que** la morpholine est l'amorolfine.

9. Composition selon la revendication 5, **caractérisée en ce que** l'allylamine peut être choisie dans le groupe comprenant la terbinafine et la tolnaftate.

10. Composition selon l'une quelconque des revendications 1 à 3, **caractérisée en ce que** le principe actif peut être un agent anti-séborrhéique, de préférence le sulfure de sélénium ou le peroxyde de benzoyle.

11. Composition selon l'une quelconque des revendications précédentes, **caractérisée en ce que** la quantité de principe actif dermatologique est comprise en poids entre 0,001% et 5%, préférentiellement entre 0,01% et 1%, plus préférentiellement encore entre 0,05% et 1%, encore plus préférentiellement environ égale à 0, 1 % du poids total.

12. Composition selon l'une quelconque des revendications précédentes, **caractérisée en ce que** la phase aqueuse comprend en outre un ou plusieurs solvants cosmétiquement acceptables pouvant être choisis dans le groupe comprenant le glycérol, le propylène glycol, le sorbitol, l'éthanol.

13. Composition selon l'une quelconque des revendications précédentes, **caractérisée en ce que** la phase aqueuse comprend en outre un ou plusieurs composés hydrosolubles pouvant être choisis dans le groupe comprenant les acides, les bases, les colorants, les agents actifs hydratants, apaisants, cicatrisants, exfoliants, antioxydants.

14. Composition selon l'une quelconque des revendications précédentes, **caractérisée en ce que** la quantité de phase aqueuse est comprise en poids entre 30% et 60%, préférentiellement entre 35% et 55% du poids total.

15. Composition selon l'une quelconque des revendications précédentes, **caractérisée en ce que** la quantité de phase grasse est comprise en poids entre 0,5% et 20%, préférentiellement entre 2% et 18%, plus préférentiellement encore entre 2,5% et 15% et encore plus préférentiellement entre 5% et 10% du poids total.

16. Composition selon l'une quelconque des revendications précédentes, **caractérisée en ce que** la phase grasse est composée de constituants pouvant être choisis dans le groupe comprenant les huiles végétales ou minérales, les triglycérides, les monoglycérides, les diglycerides, les dérivés d'acides gras, les esters d'acides gras, les huiles naturelles hydrogénées ou non, synthétiques ou non, hydrocarbonées, cycliques ou aliphatiques, linéaires ou ramifiées, saturées ou non, les silicones volatiles ou non, organomodifiées ou non, solubles ou non, les huiles perfluorées ou fluorées, les polybutènes et polyisobutènes, les esters gras liquides, utilisés seuls ou en mélange, les huiles de paraffine, de myristate d'octododécyle, de myristate d'isopropyle, de tricaprilic glycérol, de capric-caprilique glyceride.

17. Composition selon l'une quelconque des revendications précédentes, **caractérisée en ce que** le tensioactif non ionique éthoxylé peut être choisi dans le groupe comprenant les alcools gras éthoxylés, les acides gras éthoxylés, les acides gras éthoxylés ethérifiés par des groupes alkyle ou alkényle, les acides gras éthoxylés estérifiés par des groupes alkyle ou alkényle, les esters éthoxylés d'acides gras saturés ou insaturés, de glycérol, de propylène glycol, de glycol, de polyéthylène glycol, de sorbitan, les mélanges de glycérides et d'esters capriliques de polyéthylène glycol, les copolymères d'oxyde d'éthylène et d'oxyde de propylène.

18. Composition selon l'une quelconque des revendications précédentes, **caractérisée en ce que** la quantité en poids de tensioactif non ionique éthoxylé est inférieure à 20% du poids total, préférentiellement comprise entre 10% et 20% du poids total.

19. Composition selon l'une quelconque des revendications précédentes, **caractérisée en ce que** le rapport de la quantité en poids de cotensioactif stabilisant le tensioactif non ionique éthoxylé sur la quantité de tensioactif non ionique éthoxylé est compris entre 1:2 et 1:6, préférentiellement entre entre 1:2 et 1:4.

20. Composition selon l'une quelconque des revendications précédentes, **caractérisée en ce que** la quantité totale en poids de tensioactif non ionique éthoxylé et de cotensioactif stabilisant le tensioactif non ionique éthoxylé est comprise entre 15% et 40%, préférentiellement entre 20% et 35% du poids total.

21. Composition selon l'une quelconque des revendications précédentes, **caractérisée en ce que** le cotensioactif stabilisant le tensioactif non ionique éthoxylé peut être choisi dans le groupe comprenant les esters non ethoxylés d'acides gras saturés ou insaturés et de glycérol, de polyglycérol, de propylène glycol, de glycol, de polyéthylène glycol ou de sorbitan, le diisostéarate de triglycérol, l'oléate de triglycérol, le propylène glycol monolaurate, le propylène glycol monocaprylate, le monostéarate de sorbitan.

22. Composition selon l'une quelconque des revendications précédentes, **caractérisée en ce que** la quantité en poids de tensioactif non ionique hydrophile non éthoxylé est comprise 1 et 30%, de préférence entre 10% et 30%, plus préférentiellement entre 13% et 25% du poids total.

23. Composition selon l'une quelconque des revendications précédentes, **caractérisée en ce que** le tensioactif non ionique hydrophile non éthoxylé peut être chosi dans le groupe comprenant les dérivés des sucres, les esters d'acide gras et de sucrose, de saccharose ou de glucose, les dérivés de glucosides, les alkylglucosides, les alkylpolyglucosides en C₈ à C₂₂ comprenant 1 à 7 unités glucose, l'octylglucoside, le décylglucoside, le lauryglucoside, le myristylglucoside, le palmitylglucoside, le stéarylglucoside, l'oléylglucoside, le behenylglucoside, les dérivés de glucosides en C₁₀ à C₁₄ comprenant 1 à 3 unités glucose, le décylglucoside.

24. Composition selon l'une quelconque des revendications précédentes, **caractérisée en ce que** son pH est compris entre 3 et 8, préférentiellement entre 4 et 6.

25. Composition selon l'une quelconque des revendications précédentes, **caractérisée en ce que** son pH est ajusté par l'ajout d'acide lactique.

26. Composition selon l'une quelconque des revendications précédentes, **caractérisée en ce que** l'agent moussant anionique peut être choisi dans le groupe comprenant les sels de sodium, d'ammonium, d'amines, d'aminoalcools ou de magnésium des alkylsulfates, des alkyléthersulfates, des alkylamidoéthersulfates, des alkylarylpoly-éthersulfates, des alkyle monoglycérides sulftates, des alkyle monoglycérides éther sulfates, alkyle monoglycérides sulfonates, des alkylsulfonates, des alkylphosphates, des alkyle isethionates, des alkylcarboxylates, des alkyle éther carboxylates, des alkyle éther amidocarboxylates, des alkylamidesulfonates, des alkylarylsulfonates, des a-oléfine-sulfonates, des paraffine-sulfonates, des alkylsulfosuccinates, des alkyléthersulfosuccinates, des alkylamidesulfosuccinates, des alkylsulfosuccinamates, des alkylsulfoacétates, des alkylétherphosphates, des acylsarcosinates, des N-acyle aminoacides, des acyliséthionates et des N-acyltaurates.

27. Composition selon l'une quelconque des revendications précédentes, **caractérisée en ce que** la quantité en poids d'agent moussant anionique est comprise entre 15% et 25% du poids total.

28. Composition selon l'une quelconque des revendications précédentes, **caractérisée en ce qu'**elle comprend en outre au moins un agent stabilisant ou renforçateur de mousse.

29. Composition selon la revendication 28, **caractérisée en ce que** l'agent stabilisant ou renforçateur de mousse peut être choisi dans le groupe comprenant les amides d'acides gras, les alkylamides, les mono et diéthanolamides, les isopropanolamides d'acides gras et sont de préférence la diéthanolamide d'acide gras de coco.

30. Composition selon la revendication 28 ou 29, **caractérisée en ce que** la quantité en poids d'agent stabilisant ou renforçateur de mousse être comprise entre 0% et 10%, préférentiellement entre 2% et 8% du poids total.

31. Composition selon l'une quelconque des revendications précédentes, **caractérisée en ce qu'**elle comprend en outre un agent épaississant choisi parmi les agents non pontants.

32. Composition selon la revendication 31, **caractérisée en ce que** l'agent épaississant peut être choisi dans le groupe comprenant les sels de sodium, les carbomères ou les gommes xanthanes et est de préférence le chlorure de sodium ou le citrate de sodium.

33. Composition selon la revendication 31 ou 32, **caractérisée en ce que** la quantité en poids d'agent épaississant est comprise entre 0% et 4%, préférentiellement entre 1% et 3% du poids total.

34. Composition selon l'une quelconque des revendications précédentes, **caractérisée en ce qu'**elle comprend en outre un conservateur.

35. Composition selon la revendication 34, **caractérisée en ce que** le conservateur peut être choisi dans le groupe comprenant l'acide borique, l'acide benzoique, l'acide lactique, l'acide sorbique, l'alcool benzylique, le phénoxyéthanol, le chlorocrésol, les esters de l'acide parahydroxybenzoïque, les dérivés de l'imidazolidinurée et est préférentiellement l'acide sorbique et ses sels, et plus préférentiellement le sorbate de potassium.

36. Composition selon la revendication 34 ou 35, **caractérisée en ce que** le conservateur est le sorbate de potassium à une concentration en poids comprise entre 0,01 % et 0,2% du poids total.

37. Procédé de préparation d'une composition selon l'une quelconque des revendications précédentes, **caractérisé en ce qu'**il comprend les étapes successives suivantes :
- mélanger la phase grasse avec le système de tensioactifs et homogénéiser le mélange par agitation mécanique ;
- mélanger les différents adjuvants dans la phase aqueuse, comme par exemple l'agent épaississant ;
- incorporer la phase aqueuse dans la phase grasse sous agitation à une vitesse et pendant un temps suffisant pour atteindre la transparence de la composition ;
- éventuellement ajuster le pH de l'émulsion par l'ajout d'une solution acide ou basique ;
- incorporer le principe actif par agitation jusqu'à dissolution complète de celui-ci à température ambiante.

38. Composition selon l'une quelconque des revendications 1 à 36 à titre de médicament.

39. Utilisation d'une composition selon l'une quelconque des revendications 1 à 36 pour la fabrication d'un médicament destiné à traiter les troubles dermatologiques, le cuir chevelu et la dermatite séborrhéique.

40. Utilisation d'une composition selon l'une quelconque des revendications 1 à 36 en tant que cosmétique.
